# FASCICULE DE BREVET EUROPEEN

(11) **EP 2 043 594 B1**
(45) Date de publication et mention de la délivrance du brevet: **15.03.2017**
(21) Numéro de dépôt: 07823543.9
(22) Date de dépôt: 03.07.2007
(51) Int. Cl.: A61Q 17/04

(54) **UTILISATION EN COSMÉTIQUE D'UN DERIVÉ C-GLYCOSIDE EN ASSOCIATION AVEC DE L'ACIDE ASCORBIQUE**
KOSMETISCHE ANWENDUNG EINES C-GLYCOSID-DERIVATS IN KOMBINATION MIT ASCORBINSÄURE
COSMETIC USE OF A C-GLYCOSIDE DERIVATIVE IN COMBINATION WITH ASCORBIC ACID

(30) Priorité: 03.07.2006 FR 0606027; 25.07.2006 US 832941 P
(43) Date de publication de la demande: 08.04.2009
(60) Demande divisionnaire: 16188661.9
(73) Titulaire: L'Oréal, 75008 Paris (FR)
(72) Inventeur: PINEAU, Nathalie, 95220 Herblay (FR); CATROUX, Philippe, 33230 Guitres (FR)
(74) Mandataire: Nony
(86) Numéro de dépôt international: PCT/FR2007/051587
(87) Numéro de publication internationale: WO 2008/003900

(56) Documents cités:
- EP-A- 1 589 010
- WO-A2-02/051803
- WO-A2-02/051828
- WO-A2-2004/028483
- US-A1- 2005 250 708

## Description

La présente invention se rapporte à l'utilisation, dans le domaine des compositions cosmétiques et/ou dermatologiques, d'une association synergique d'au moins l'acide ascorbique à au moins un dérivé C-glycoside choisi parmi le C-β-D-xylopyranoside-2-hydroxy-propane, le C-α- D-xylopyranoside-2-hydroxy-propane, et leurs mélanges. Elle vise en outre des compositions cosmétiques et/ou dermatologiques comprenant une telle association.

Ces associations et compositions sont préférentiellement destinées à prévenir et/ou traiter les signes cutanés du vieillissement et/ou du photovieillissement de la peau et en particulier à augmenter la synthèse de collagène.

La peau humaine est constituée de deux tissus l'un superficiel, l'épiderme, l'autre profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac ...), appelé «fonction barrière ».

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses.

La cohésion entre l'épiderme et le derme est assurée par la jonction dermo-épidermique. C'est une région complexe d'une épaisseur de 100 nm environ qui comprend le pôle basal des kératinocytes basaux, la membrane épidermique et la zone sous basale du derme superficiel (Bernard P. Structure de la jonction dermo-épidermique. Objectif peau. 2001, 68 : 87-93). D'un point de vue structurel, des hémidesmosomes, dans lesquels s'insèrent des filaments de kératine (complexe hémidesmosome-tonofilaments), sont répartis sur la membrane plasmique des kératinocytes basaux. Au regard de ces complexes hémidesmosome-tonofilaments, on trouve des filaments d'ancrage qui traversent la membrane basale épidermique. Les filaments d'ancrage sont reliés à la laminine 5 côté épidermique. Enfin, des fibrilles d'ancrage constituent le réseau sous basal. Ce sont des structures curvilinéaires qui prennent naissance et se terminent sur la face profonde de la membrane basale et dans lesquelles viennent s'engager des fibres de collagène I, III et V. Il a été montré que ces fibrilles d'ancrage, parfaitement visualisables en microscopie électronique, sont composées de collagène de type VII (ci-après collagène VII). Le collagène VII est synthétisé par les kératinocytes et les fibroblastes mais de façon plus importante par les kératinocytes (Aumailley M, Rousselle P. laminins of the dermoepdiermal junction. Matrix Biology. 1999, 18 : 19-28 ; Nievers M, Schaapveld R, Sonnenberg A. Biology and function of hemidesmosomes, Matrix Biology, 1999, 18 : 5-17.

Ainsi, les collagènes sont les protéines majoritaires des matrices extracellulaires. A ce jour, 20 types de collagènes sont identifiés et notés de I à XX. On distingue différentes familles de collagène parmi ces types en fonction des structures formées :
- la famille des collagènes fibrillaires (type I, II, II V/XI) qui forment donc des fibres ;
- la famille des collagènes formant le réseau des 5 membranes basales qui comprend le collagène de type VI et de type XVII ;
- les collagènes formant des réseaux hexagonaux (type VIII et type X), des filaments perlés (Type VI), des FACIT (type IX, XII, XIV, XVI, XIX, XX) ;
- les fibrilles d'ancrage qui correspondent au type VII ;
- les multiplexines (type XV, XVII) et
- le collagène de type XIII dont on ne connaît pas les fonctions précises à ce jour.

Dans la peau, les collagènes majoritairement présents dans l'ensemble du derme sont les collagènes de type I et III qui forment la matrice extracellulaire de l'ensemble du derme (ce sont ces collagènes qui constituent 70-80 % du poids sec du derme).

Par ailleurs, les collagènes ne sont pas tous synthétisés par les mêmes types cellulaires, les collagènes de type I et III sont essentiellement produits par le fibroblaste dermique alors que le collagène de type VII est produit par le kératinocyte épidermique. Enfin, la régulation de leur expression diffère d'un collagène à un autre, par exemple, les collagènes I et VII ne sont pas régulés de la même manière par certaines cytokines, en effet, le TNF alpha et la leukoreguline stimulent le collagène VII et régulent négativement le collagène I.

Enfin, toutes les molécules de collagène sont des variantes d'un précurseur commun qui est la chaîne α du procollagène.

Avec le vieillissement, le collagène s'amincit et des rides apparaissent à la surface de la peau. Le vieillissement cutané est un mécanisme génétiquement programmé. Par ailleurs, certains facteurs d'environnement comme le tabagisme et surtout l'exposition au rayonnement du soleil l'accélèrent. La peau a ainsi un aspect beaucoup plus âgé sur les zones exposées au soleil comme le dos des mains ou le visage. Ainsi, ces autres facteurs ont également un impact négatif sur le collagène naturel de la peau.

En conséquence, au regard du rôle déterminant du collagène et plus particulièrement du collagène de type VII, au niveau de la cohésion entre les tissus épiderme et derme, et par voie de conséquence au niveau de l'intégrité de la peau et de sa résistance aux agressions externes de type mécaniques, la stimulation de synthèse de ces différentes formes de collagène apparaît comme un moyen efficace pour pallier les signes du vieillissement cutané.

La présente invention a précisément pour but de proposer une nouvelle association susceptible d'être utilisée dans le domaine cosmétique pour limiter le vieillissement de la peau, et notamment le vieillissement généré par une diminution de l'élasticité de la peau et/ou par une dégradation du collagène dans la structure des tissus.

De façon surprenante, les inventeurs ont constaté qu'une association entre l'acide ascorbique et un dérivé C-glycoside selon l'invention était capable d'augmenter la synthèse de procollagène 1 et ainsi de contrer les signes du vieillissement.

Les sucres et dérivés du sucre sont des produits déjà mis à profit à des fins diverses pour la formulation de compositions cosmétiques destinées tant au soin de la peau qu'au soin et/ou lavage des fibres kératiniques. Ainsi, dans WO 99/24009, le D-xylose et ses dérivés sont proposés à des fins de préparation de produits cosmétiques ou pharmaceutiques visant à améliorer la fonctionnalité des cellules de l'épiderme.

Parmi les sucres utilisables dans le domaine, les dérivés C-glycosides s'avèrent tout particulièrement intéressants. Ainsi, certains dérivés C-glycosides ont démontré des propriétés biologiques intéressantes, en particulier pour lutter contre le vieillissement de l'épiderme et/ou contre le dessèchement de la peau. De tels composés sont notamment décrits dans le document WO 02/051828.

Ces composés sont plus particulièrement représentés par la formule : 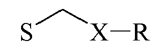 dans laquelle S représente un monosaccharide ou un polysaccharide, R représente différents radicaux linéaires ou cycliques et le groupement X peut représenter un groupement choisi parmi : -CO-, -CH(NR₁R₂)-, CHR'-, -C(=CHR')- avec R₁, R₂ et R' pouvant représenter différent radicaux, dont le radical hydroxyle.

En conséquence, un premier aspect de l'invention concerne l'utilisation cosmétique d'une association synergique d'au moins de l'acide ascorbique avec au moins un dérivé C-glycoside choisi parmi le C-β-D-xylopyranoside-2-hydroxy-propane, le C-α-D-xylopyranoside-2-hydroxy-propane, et leurs mélanges pour traiter, de manière préventive ou curative, les signes du vieillissement de la peau du corps ou du visage, choisis parmi les rides et/ou ridules, la peau flétrie, le manque d'élasticité et/ou de tonus de la peau, l'amincissement du derme, la dégradation des fibres de collagène, la peau molle et la peau amincie.

Le présent texte décrit également l'utilisation d'une association synergique selon l'invention pour traiter, de manière préventive ou curative, les signes du vieillissement de la peau du corps ou du visage, qu'ils soient chronobiologiques ou photo-induits, et notamment le vieillissement généré par une diminution de l'élasticité de la peau et/ou par une dégradation du collagène dans la structure des tissus.

Le présent texte décritl'utilisation d'une association telle que définie ci-dessus, pour traiter, de manière préventive ou curative, les rides et/ou ridules, la peau flétrie, le manque d'élasticité et/ou de tonus de la peau, l'amincissement du derme, la dégradation des fibres de collagène, la peau molle, la peau amincie, les dégradations internes de la peau consécutives à une exposition aux rayonnements ultra-violets.

Un autre objet de l'invention est l'utilisation d'une association telle que définie ci-dessus, pour inhiber l'activité des élastases et/ou pour limiter et/ou pour combattre la dégradation des fibres élastiques.

Selon un autre de ses aspects, la présente invention concerne une composition cosmétique et/ou dermatologique comprenant dans un milieu physiologiquement acceptable (i) au moins un dérivé C-glycoside choisi parmi le C-β-D-xylopyranoside-2-hydroxy-propane, le C-α-D-xylopyranoside-2-hydroxy-propane, et leurs mélanges, en association avec (ii) au moins de l'acide ascorbique.

Le présent texte décrit également une composition cosmétique et/ou dermatologique comprenant dans un milieu physiologiquement acceptable contenant une phase aqueuse, au moins un dérivé C-glycoside selon l'invention en association avec au moins de l'acide ascorbique.

Le présent texte décrit une composition cosmétique et/ou dermatologique anhydre comprenant dans un milieu physiologiquement acceptable au moins un dérivé C-glycoside en association avec au moins de l'acide ascorbique, l'un de ses dérivés ou analogues.

Un autre objet de l'invention est un procédé de traitement cosmétique de la peau du corps ou du visage, y compris le cuir chevelu, dans lequel on applique sur la peau une composition cosmétique telle que définie ci-dessus.

Le présent texte décrit l'utilisation d'une association telle que définie ci-dessus pour la préparation d'une composition dermatologique destinée à prévenir et/ou traiter les signes de vieillissement cutané et/ou à stimuler la synthèse du collagène, en particulier du procollagène I.

Le présent texte décrit également un procédé de traitement thérapeutique ou non, et en particulier cosmétique, destiné à prévenir et/ou traiter les signes du vieillissement cutané comprenant l'administration à un sujet d'une association ou d'une composition telle que définie ci-dessus.

Comme il ressort des exemples soumis ci-après, les inventeurs ont constaté que l'efficacité d'une association conforme à l'invention s'avère contre toute attente nettement supérieure à celle attendue de la simple addition des effets respectifs de chacun des deux composés.

Par signes cutanés du vieillissement on entend toutes modifications de l'aspect extérieur de la peau dues au vieillissement qu'il soit chronobiologique et/ou photo-induit, comme par exemple les rides et ridules, la peau flétrie, le manque d'élasticité et/ou de tonus de la peau, l'amincissement du derme et/ou la dégradation des fibres de collagène ce qui entraîne l'apparence de peau molle et ridée; on entend également toutes les modifications internes de la peau qui ne se traduisent pas systématiquement par un aspect extérieur modifié, comme par exemple toutes dégradations internes de la peau, particulièrement des fibres d'élastine, ou fibres élastiques, consécutives à une exposition aux rayonnements ultra-violets.

### DERIVES C-GLYCOSIDE

Un dérivé C-glycoside décrit dans le présent texte peut être un composé de formule générale (I) suivante : 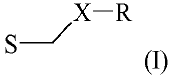 dans laquelle :
- R représente :
   - un radical alkyle linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀, en particulier en C₃ à C₁₀ ;
   - un radical hydrofluoro- ou perfluoro-alkyle, linéaire saturé en C₁ à C₂₀, en particulier en C₁ à C₁₀, ou insaturé en C₂ à C₂₀, en particulier en C₂ à C₁₀, ou ramifié ou cyclique, saturé ou insaturé, en C₃ à C₂₀, en particulier en C₃ à C₁₀ ;
   la chaîne hydrocarbonée constituant lesdits radicaux pouvant, le cas échéant, être interrompue par 1, 2, 3 ou plus d'hétéroatomes choisis parmi :
   - un oxygène,
   - un soufre,
   - un azote, et
   - un silicium,
   et pouvant être éventuellement substituée par au moins un radical choisi parmi :
   - -OR₄,
   - -SR₄,
   - -NR₄R₅,
   - -COOR₄,
   - -CONHR₄,
   - -CN,
   - un atome d'halogène,
   - un radical hydrofluoro- ou perfluoro-alkyle, en C₁ à C₆, et/ou
   - un radical cycloalkyle en C₃ à C₈,
   avec R₄ et R₅ pouvant représenter, indépendamment l'un de l'autre, un atome d'hydrogène, ou un radical alkyle, perfluoroalkyle ou hydrofluoroalkyle linéaire, saturé en C₁ à C₃₀, notamment en C₁ à C₁₂, ou insaturé en C₂ à C₃₀, notamment en C₂ à C₁₂, ou ramifié ou cyclique, saturé ou insaturé, en C₃ à C₃₀, notamment en C₃ à C₁₂ ; ou un radical aryle en C₆ à C₁₀,
- X représente un radical choisi parmi les groupements : 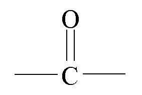 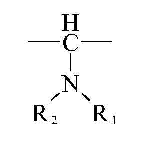 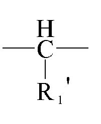 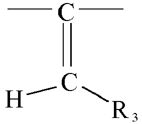 avec R₁, R₂ et R₃ représentant, indépendamment l'un de l'autre, un atome d'hydrogène, ou un radical R, avec R tel que défini précédemment, et R'₁ représente un atome d'hydrogène, un groupe -OH ou un radical R tel que défini précédemment, R₁ pouvant désigner également un radical aryle en C₆ à C₁₀ ;
   - S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
   - la liaison S-CH₂-X représente une liaison de nature C-anomérique, qui peut être α ou β,
   ainsi que leurs sels cosmétiquement acceptables, leurs solvates tels que les hydrates et leurs isomères.

Dans le présent texte, par « halogène », on entend le chlore, le fluor, le brome ou l'iode.

Le terme « aryle » désigne un cycle aromatique tel que phényle, éventuellement substitué par un ou plusieurs radicaux alkyles en C₁-C₄.

Le terme « cycloalkyle en C₃ à C₈ » désigne un cycle aliphatique ayant de 3 à 8 atomes de carbone, incluant par exemple le cyclopropyle, le cyclopentyle et le cyclohéxyle.

Parmi les groupes alkyle mentionnés dans le présent texte, on peut notamment citer les groupes méthyle, éthyle, isopropyle, n-propyle, n-butyle, t-butyle, isobutyle, sec-butyle, pentyle, n-hexyle, cyclopropyle, cyclopentyle, cyclohexyle, et allyle.

Le présent texte décrit l'utilisation d'un dérivé C-glycoside répondant à la formule (I) pour lequel S peut représenter un monosaccharide ou un polysaccharide contenant jusqu'à 6 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine obligatoirement protégée, X et R conservant par ailleurs l'ensemble des définitions précédemment données.

Avantageusement, un monosaccharide peut être choisi parmi le D-glucose, le D-galactose, le D-mannose, le D-xylose, le D-lyxose, le L-fucose, le L-arabinose, le L-rhamnose, l'acide D-glucuronique, l'acide D-galacturonique, l'acide D-iduronique, la N-acétyl-D-glucosamine, la N-acétyl-D-galactosamine et désigne avantageusement le D-glucose, le D-xylose, la N-acétyl-D-glucosamine ou le L-fucose, et en particulier le D-xylose.

Plus particulièrement, un polysaccharide contenant jusqu'à 6 unités sucre peut être choisi parmi le D-maltose, le D-lactose, le D-cellobiose, le D-maltotriose, un disaccharide associant un acide uronique choisi parmi l'acide D-iduronique ou l'acide D-glucuronique avec une hexosamine choisie parmi la D-galactosamine, la D-glucosamine, la N-acétyl-D-galactosamine, la N-acétyl-D-glucosamine, un oligosaccharide contenant au moins un xylose qui peut être avantageusement choisi parmi le xylobiose, le méthyl-β-xylobioside, le xylotriose, le xylotétraose, le xylopentaose et le xylohexaose et notamment le xylobiose qui est composé de deux molécules de xylose liées par une liaison 1-4.

Plus particulièrement, S peut représenter un monosaccharide choisi parmi le D-glucose, le D-xylose, le L-fucose, le D-galactose, le D-maltose et notamment le D-xylose.

Selon le présent texte, on peut utiliser des dérivés C-glycoside répondant à la formule (I) pour lesquels X représente un groupement choisi parmi -CO-, -CH(OH)-, -CH(NR₁R₂)-, -CH(R)-, en particulier -CO-, -CH(OH)-, -CH(NH₂)-, -CH(NHCH₂CH₂CH₂OH)-, -CH(NHPh)-, -CH(CH₃)-, et plus particulièrement un groupement -CO-, -CH(OH)-, -CH(NH₂)-, et préférentiellement un groupement-CH(OH)-, S et R conservant par ailleurs l'ensemble des définitions précédemment données.

Selon le présent texte, on peut utiliser un dérivé C-glycoside répondant à la formule (I) pour lesquels R représente un radical alkyle linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀ ; en particulier en C₃ à C₁₀, et éventuellement substitué comme décrit précédemment, S et X conservant par ailleurs l'ensemble des définitions précédemment données. De préférence, R désigne un radical linéaire en C₁-C₄, notamment C₁-C₃, éventuellement substitué par -OH, -COOH ou-COOR"₂, R"₂ étant un radical alkyle saturé en C₁-C₄, notamment éthyle. Préférentiellement R désigne un radical alkyle linéaire non substitué en C₁-C₄, notamment C₁-C₂, en particulier éthyle.

Parmi les dérivés C-glycoside de formule (I), on utilise de préférence ceux pour lesquels :
- R représente un radical alkyle en linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀ ; en particulier en C₃ à C₁₀, et éventuellement substitué comme décrit précédemment ;
- S représente un monosaccharide comme décrit précédemment ;
- X représente -CO-, -CH(OH)-, -CH(NR₁R₂)-, -CH(R)- comme décrit précédemment.

De préférence, on utilise un dérivé C-glycoside de formule (I) pour lesquels :
- R désigne un radical linéaire en C₁-C₄, notamment C₁-C₃, éventuellement substitué par -OH, -COOH ou -COOR"₂, R"₂ étant un radical alkyle saturé en C₁-C₄, notamment éthyle ;
- S représente un monosaccharide comme décrit précédemment ;
- X représente un groupement choisi parmi -CO-, -CH(OH)-, -CH(NH₂)-, -CH(NHCH₂CH₂CH₂OH)-, -CH(NHPh)-, -CH(CH₃)-, et plus particulièrement un groupement -CO-, -CH(OH)-, -CH(NH₂)-,et préférentiellement un groupement -CH(OH)-.

Préférentiellement, on utilise un dérivé C-glycoside de formule (I) pour lesquels :
- R désigne un radical alkyle linéaire non substitué en C₁-C₄, notamment C₁-C₂, en particulier éthyle ;
- S représente un monosaccharide comme décrit précédemment ; notamment le D-glucose, le D-xylose, la N-acétyl-D-glucosamine ou le L-fucose, et en particulier le D-xylose ;
- X représente un groupement choisi parmi -CO-, -CH(OH)-, -CH(NH₂)-, et préférentiellement un groupement -CH(OH)-.

Les sels acceptables pour l'usage non thérapeutique des composés décrits dans le présent texte comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque le composé de formule (I) comporte un groupe acide, la neutralisation du ou des groupes acides peut être effectuée par une base minérale, telle que LiOH, NaOH, KOH, Ca(OH)₂, NH₄OH, Mg(OH)₂ ou Zn(OH)₂ ; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans le présent texte comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Parmi les dérivés C-glycoside de formule (I) selon le présent texte, on décrit tout particulièrement :
1. C-β-D-xylopyranoside-n-propane-2-one ;
2. C-α-D-xylopyranoside-n-propane-2-one ;
3. 1-[2-(3-hydroxy-propylamino)-propyl]- C- P -D-xylopyranose ;
4. 1-[2-(3-hydroxy-propylamino)-propyl]-C-α-D-xylopyranose ;
5. C-β-D-xylopyranoside-2-hydroxy-propane ;
6. C-α-D-xylopyranoside-2-hydroxy-propane ;
7. C-β-D-xylopyranoside-2-amino-propane ;
8. C-α-D-xylopyranoside-2-amino-propane ;
9. C-β-D-xylopyranoside-2-phénylamino-propane ;
10. C-α-D-xylopyranoside-2-phénylamino-propane ;
11. ester éthylique de l'acide 3-méthyl-4-(C-β-D-xylopyranoside)-butyrique ;
12. ester éthylique de l'acide 3-méthyl-4-(C-α-D-xylopyranoside)-butyrique ;
13. acide 6-(C-β-D-xylopyranoside)-5-céto-hexanoique ;
14. acide 6-(C-α-D-xylopyranoside)-5-céto-hexanoique ;
15. acide 6-(C-β-D-xylopyranoside)-5-hydroxy-hexanoique ;
16. acide 6-(C-α-D-xylopyranoside)-5-hydroxy-hexanoique ;
17. acide 6-(C-β-D-xylopyranoside)-5-amino-hexanoique ;
18. acide 6-(C-α-D-xylopyranoside)-5-amino-hexanoique ;
19. acide 6-(C-β-D-xylopyranoside)-5-phénylamino-hexanoique ;
20. acide 6-(C-α-D-xylopyranoside)-5-phénylamino-hexanoique ;
21. 1-(C-β-D-xylopyranoside)-hexane-2,6-diol ;
22. 1-(C-α-D-xylopyranoside)-hexane-2,6-diol ;
23. acide 5-(C-β-D-xylopyranoside)-4-céto-pentanoique ;
24. acide 5-(C-α-D-xylopyranoside)-4-céto-pentanoique ;
25. acide 5-(C-β-D-xylopyranoside)-4-hydroxy-pentanoique ;
26. acide 5-(C-α-D-xylopyranoside)-4-hydroxy-pentanoique ;
27. acide 5-(C-β-D-xylopyranoside)-4-amino-pentanoique ;
28. acide 5-(C-α-D-xylopyranoside)-4-amino-pentanoique ;
29. acide 5-(C-β-D-xylopyranoside)-4-phénylamino-pentanoique ;
30. acide 5-(C-α-D-xylopyranoside)-4-phénylamino-pentanoique ;
31. 1-(C-β-D-xylopyranoside)-pentane-2,5-diol ;
32. 1-(C-α-D-xylopyranoside)-pentane-2,5-diol ;
33. 1-(C-β-D-fucopyranoside)-propane-2-one ;
34. 1-(C-α-D-fucopyranoside)-propane-2-one ;
35. 1-(C-β-L-fucopyranoside)-propane-2-one ;
36. 1-(C-α-L-fucopyranoside)-propane-2-one ;
37. 1-(C-β-D-fucopyranoside)-2-hydroxy-propane ;
38. 1-(C-α-D-fucopyranoside)-2-hydroxy-propane ;
39. 1-(C-β-L-fucopyranoside)-2-hydroxy-propane ;
40. 1-(C-α-L-fucopyranoside)-2-hydroxy-propane ;
41. 1-(C-β-D-fucopyranoside)-2-amino-propane ;
42. 1-(C-α-D-fucopyranoside)-2-amino-propane ;
43. 1-(C-β-L-fucopyranoside)-2-amino-propane ;
44. 1-(C-α-L-fucopyranoside)-2-amino-propane ;
45. 1-(C-β-D-fucopyranoside)-2-phénylamino-propane ;
46. 1-(C-α-D-fucopyranoside)-2-phénylamino-propane ;
47. 1-(C-β-L-fucopyranoside)-2-phénylamino-propane ;
48. 1-(C-α-L-fucopyranoside)-2-phénylamino-propane ;
49. ester éthylique de l'acide 3-méthyl-4-(C-β-D-fucopyranoside)-butyrique ;
50. ester éthylique de l'acide 3-méthyl-4-(C-α-D-fucopyranoside)-butyrique ;
51. ester éthylique de l'acide 3-méthyl-4-(C-β-L-fucopyranoside)-butyrique ;
52. ester éthylique de l'acide 3-méthyl-4-(C-α-L-fucopyranoside)-butyrique ;
53. acide 6-(C-β-D-fucopyranoside)-5-céto-hexanoique ;
54. acide 6-(C-α-D-fucopyranoside)-5-céto-hexanoique ;
55. acide 6-(C-β-L-fucopyranoside)-5-céto-hexanoique ;
56. acide 6-(C-α-L-fucopyranoside)-5-céto-hexanoique ;
57. acide 6-(C-β-D-fucopyranoside)-5-hydroxy-hexanoique ;
58. acide 6-(C-α-D-fucopyranoside)-5-hydroxy-hexanoique ;
59. acide 6-(C-β-L-fucopyranoside)-5-hydroxy-hexanoique ;
60. acide 6-(C-α-L-fucopyranoside)-5-hydroxy-hexanoique ;
61. acide 6-(C-β-D-fucopyranoside)-5-amino-hexanoique ;
62. acide 6-(C-α-D-fucopyranoside)-5-amino-hexanoique ;
63. acide 6-(C-β-L-fucopyranoside)-5-amino-hexanoique ;
64. acide 6-(C-α-L-fucopyranoside)-5-amino-hexanoique ;
65. 1-(C-β-D-fucopyranoside)-hexane-2,6-diol ;
66. 1-(C-α-D-fucopyranoside)-hexane-2,6-diol ;
67. 1-(C-β-L-fucopyranoside)-hexane-2,6-diol ;
68. 1-(C-α-L-fucopyranoside)-hexane-2,6-diol ;
69. acide 5-(C-β-D-fucopyranoside)-4-céto-pentanoique ;
70. acide 5-(C-α-D-fucopyranoside)-4-céto-pentanoique ;
71. acide 5-(C-β-L-fucopyranoside)-4-céto-pentanoique ;
72. acide 5-(C-α-L-fucopyranoside)-4-céto-pentanoique ;
73. acide 5-(C-β-D-fucopyranoside)-4-hydroxy-pentanoique ;
74. acide 5-(C-α-D-fucopyranoside)-4-hydroxy-pentanoique ;
75. acide 5-(C-β-L-fucopyranoside)-4-hydroxy-pentanoique ;
76. acide 5-(C-α-L-fucopyranoside)-4-hydroxy-pentanoique ;
77. acide 5-(C-β-D-fucopyranoside)-4-amino-pentanoique ;
78. acide 5-(C-α-D-fucopyranoside)-4-amino-pentanoique
79. acide 5-(C-β-L-fucopyranoside)-4-amino-pentanoique ;
80. acide 5-(C-α-L-fucopyranoside)-4-amino-pentanoique ;
81. 1-(C-β-D-fucopyranoside)-pentane-2,5-diol ;
82. 1-(C-α-D-fucopyranoside)-pentane-2,5-diol ;
83. 1-(C-β-L-fucopyranoside)-pentane-2,5-diol ;
84. 1-(C-α-L-fucopyranoside)-pentane-2,5-diol ;
85. 1-(C-β-D-glucopyranosyl)-2-hydroxy-propane ;
86. 1-(C-α-D-glucopyranosyl)-2-hydroxy-propane ;
87. 1-(C-β-D-glucopyranosyl)-2-amino-propane ;
88. 1-(C-α-D-glucopyranosyl)-2-amino-propane ;
89. 1-(C-β-D-glucopyranosyl)-2-phénylamino-propane ;
90. 1-(C-α-D-glucopyranosyl)-2-phénylamino-propane ;
91. ester éthylique de l'acide 3-méthyl-4-(C-β-D-glucopyranosyl)-butyrique ;
92. ester éthylique de l'acide 3-méthyl-4-(C-α-D-glucopyranosyl)-butyrique ;
93. acide 6-(C-β-D-glucopyranosyl)-5-céto-hexanoique ;
94. acide 6-(C-α-D-glucopyranosyl)-5-céto-hexanoique ;
95. acide 6-(C-β-D-glucopyranosyl)-5-hydroxy-hexanoique ;
96. acide 6-(C-α-D-glucopyranosyl)-5-hydroxy-hexanoique ;
97. acide 6-(C-β-D-glucopyranosyl)-5-amino-hexanoique ;
98. acide 6-(C-α-D-glucopyranosyl)-5-amino-hexanoique ;
99. acide 6-(C-β-D-glucopyranosyl)-5-phénylamino-hexanoique ;
100. acide 6-(C-α-D-glucopyranosyl)-5-phénylamino-hexanoique ;
101. 1-(C-β-D-glucopyranosyl)-hexane-2,6-diol ;
102. 1-(C-α-D-glucopyranosyl)-hexane-2,6-diol ;
103. acide 6-(C-β-D-glucopyranosyl)-5-céto-pentanoique ;
104. acide 6-(C-α-D-glucopyranosyl)-5-céto-pentanoique ;
105. acide 6-(C-β-D-glucopyranosyl)-5-hydroxy-pentanoique ;
106. acide 6-(C-α-D-glucopyranosyl)-5-hydroxy-pentanoique ;
107. acide 6-(C-β-D-glucopyranosyl)-5-amino-pentanoique ;
108. acide 6-(C-α-D-glucopyranosyl)-5-hydroxy-pentanoique ;
109. acide 6-(C-β-D-glucopyranosyl)-5-phénylamino-pentanoique ;
110. acide 6-(C-α-D-glucopyranosyl)-5-phénylamino-pentanoique ;
111. 1-(C-β-D-glucopyranosyl)-pentane-2,5-diol ;
112. 1-(C-α-D-glucopyranosyl)-pentane-2,5-diol ;
113. 1-(C-β-D-galactopyranosyl)-2-hydroxy-propane ;
114. 1-(C-α-D-galactopyranosyl)-2-hydroxy-propane ;
115. 1-(C-β-D-galactopyranosyl)-2-amino-propane ;
116. 1-(C-α-D-galactopyranosyl)-2-amino-propane ;
117. 1-(C-β-D-galactopyranosyl)-2-phénylamino-propane ;
118. 1-(C-α-D-galactopyranosyl)-2-phénylamino-propane ;
119. ester éthylique de l'acide 3-méthyl-4-(β-D-galactopyranosyl)-butyrique ;
120. ester éthylique de l'acide 3-méthyl-4-(α-D-galactopyranosyl)-butyrique ;
121. acide 6-(C-β-D-galactopyranosyl)-5-céto-hexanoique ;
122. acide 6-(C-α-D-galactopyranosyl)-5-céto-hexanoique ;
123. acide 6-(C-β-D-galactopyranosyl)-5-hydroxy-hexanoique ;
124. acide 6-(C-α-D-galactopyranosyl)-5-hydroxy-hexanoique ;
125. acide 6-(C-β-D-galactopyranosyl)-5-amino-hexanoique ;
126. acide 6-(C-α-D-galactopyranosyl)-5-amino-hexanoique ;
127. acide 6-(C-β-D-galactopyranosyl)-5-phénylamino-hexanoique ;
128. acide 6-(C-α-D-galactopyranosyl)-5-phénylamino-hexanoique ;
129. 1-(C-β-D-galactopyranosyl)-hexane-2,6-diol ;
130. 1-(C-α-D-galactopyranosyl)-hexane-2,6-diol ;
131. acide 6-(C-β-D-galactopyranosyl)-5-céto-pentanoique ;
132. acide 6-(C-α-D-galactopyranosyl)-5-céto-pentanoique ;
133. acide 6-(C-β-D-galactopyranosyl)-5-hydroxy-pentanoique ;
134. acide 6-(C-α-D-galactopyranosyl)-5-hydroxy-pentanoique ;
135. acide 6-(C-β-D-galactopyranosyl)-5-amino-pentanoique ;
136. acide 6-(C-α-D-galactopyranosyl)-5-amino-pentanoique ;
137. acide 6-(C-β-D-galactopyranosyl)-5-phénylamino-pentanoique ;
138. acide 6-(C-α-D-galactopyranosyl)-5-phénylamino-pentanoique ;
139. 1-(C-β-D-galactopyranosyl)-pentane-2,6-diol ;
140. 1-(C-α-D-galactopyranosyl)-pentane-2,6-diol ;
141. 1-(C-β-D-fucofuranosyl)-propane-2-one ;
142. 1-(C-α-D-fucofuranosyl)-propane-2-one ;
143. 1-(C-β-L-fucofuranosyl)-propane-2-one ;
144. 1-(C-α-L-fucofuranosyl)-propane-2-one ;
145. 3'-(acétamido-C-β-D-glucopyranosyl)-propane-2'-one ;
146. 3'-(acétamido-C-α-D-glucopyranosyl)-propane-2'-one ;
147. 1-(acétamido-C-β-D-glucopyranosyl)-2-hydroxyl-propane ;
148. 1-(acétamido-C-β-D-glucopyranosyl)-2-amino-propane ;
149. 1-(acétamido-C-β-D-glucopyranosyl)-2-phénylamino-propane ;
150. 1-(acétamido-C-α-D-glucopyranosyl)-2-phénylamino-propane ;
151. ester éthylique de l'acide 3-méthyl-4-(acétamido-C-β-D-glucopyranosyl)-butyrique ;
152. ester éthylique de l'acide 3-méthyl-4-(acétamido-C-α-D-glucopyranosyl)-butyrique ;
153. acide 6-(acétamido-C-β-D-glucopyranosyl)-5-céto-hexanoique ;
154. acide 6-(acétamido-C-α-D-glucopyranosyl)-5-céto-hexanoique ;
155. acide 6-(acétamido-C-β-D-glucopyranosyl)-5-hydroxy-hexanoique ;
156. acide 6-(acétamido-C-α-D-glucopyranosyl)-5-hydroxy-hexanoique ;
157. acide 6-(acétamido-C-β-D-glucopyranosyl)-5-amino-hexanoique ;
158. acide 6-(acétamido-C-α-D-glucopyranosyl)-5-amino-hexanoique ;
159. acide 6-(acétamido-C-β-D-glucopyranosyl)-5-phénylamino-hexanoique ;
160. acide 6-(acétamido-C-α-D-glucopyranosyl)-5-phénylamino-hexanoique ;
161. 1-(acétamido-C-β-D-glucopyranosyl)-hexane-2,6-diol ;
162. 1-(acétamido-C-α-D-glucopyranosyl)-hexane-2,6-diol ;
163. acide 6-(acétamido-C-β-D-glucopyranosyl)-5-céto-pentanoique ;
164. acide 6-(acétamido-C-α-D-glucopyranosyl)-5-céto-pentanoique ;
165. acide 6-(acétamido-C-β-D-glucopyranosyl)-5-hydroxy-pentanoique ;
166. acide 6-(acétamido-C-α-D-glucopyranosyl)-5-hydroxy-pentanoique ;
167. acide 6-(acétamido-C-β-D-glucopyranosyl)-5-amino-pentanoique ;
168. acide 6-(acétamido-C-α-D-glucopyranosyl)-5-amino-pentanoique ;
169. acide 6-(acétamido-C-β-D-glucopyranosyl)-5-phénylamino-pentanoique ;
170. acide6-(acétamido-C-α-D-glucopyranosyl)-5-phénylamino-pentanoique ;
171. 1-(acétamido-C-β-D-glucopyranosyl)-pentane-2,5-diol ;
172. 1-(acétamido-C-α-D-glucopyranosyl)-pentane-2,5-diol.

A titre illustratif et non limitatif des dérivés C-glycoside décrit dans le présent texte, on peut notamment citer les dérivés suivants :
- le C-β-D-xylopyranoside-n-propane-2-one,
- le C-α- D-xylopyranoside-n-propane-2-one,
- le C-β-D-xylopyranoside-2-hydroxy-propane,
- le C-α- D-xylopyranoside-2-hydroxy-propane,
- la 1-(C-β-D-fucopyranoside)-propane-2-one,
- la 1-(C-α-D-fucopyranoside)-propane-2-one,
- la 1-(C-β-L-fucopyranoside)-propane-2-one,
- la 1-(C-α-L-fucopyranoside)-propane-2-one,
- le 1-(C-β-D-fucopyranoside)-2-hydroxy-propane,
- le 1-(C-α-D-fucopyranoside)-2-hydroxy-propane,
- le 1-(C-β-L-fucopyranoside)-2-hydroxy-propane,
- le 1-(C-α-L-fucopyranoside)-2-hydroxy-propane,
- le 1-(C-β-D-glucopyranosyl)-2-hydroxyl-propane,
- le 1-(C-α-D-glucopyranosyl)-2-hydroxyl-propane,
- le 1-(C-β-D-galactopyranosyl)-2-hydroxyl-propane,
- le 1-(C-α-D-galactopyranosyl)-2-hydroxyl-propane
- la 1-(C-β-D-fucofuranosyl)-propane-2-one,
- la 1-(C-α-D-fucofuranosyl)-propane-2-one
- la 1-(C-β-L-fucofuranosyl)-propane-2-one,
- la 1-(C-α-L-fucofuranosyl)-propane-2-one,
- le C-β-D-maltopyranoside-n-propane-2-one,
- le C-α-D-maltopyranoside-n-propane-2-one
- le C-β-D-maltopyranoside-2-hydroxy-propane,
- le C-α-D-maltopyranoside-2-hydroxy-propane, leurs isomères et leurs mélanges.

Selon un mode de réalisation, le C-β-D-xylopyranoside-2-hydroxy-propane ou le C-α-D-xylopyranoside-2-hydroxy-propane, et mieux le C-β-D-xylopyranoside-2-hydroxy-propane, peuvent être avantageusement mis en oeuvre pour la préparation d'une composition selon l'invention.

Selon un mode de réalisation particulier, le dérivé C-glycoside peut être le C-β-D-xylopyranoside-2-hydroxy-propane sous forme pure ou d'une solution à 30 % en poids en matière active dans un mélange eau/propylène glycol (60/40 % en poids) tel que le produit fabriqué par CHIMEX sous la dénomination commerciale « MEXORYL SBB^{®} ».

Bien entendu, selon le présent texte, un dérivé C-glycoside répondant à la formule (I) peut être utilisé seul ou en mélange avec d'autres dérivés C-glycosides et en toutes proportions.

Un dérivé de C-glycoside convenant à l'invention peut notamment être obtenu par la méthode de synthèse décrite dans le document WO 02/051828.

La quantité de dérivé C-glycoside à mettre en oeuvre dans une composition selon l'invention dépend de l'effet cosmétique ou dermatologique recherché, et peut donc varier dans une large mesure.

L'homme de l'art peut aisément, sur la base de ses connaissances générales, déterminer les quantités appropriées.

Une composition selon l'invention peut comprendre un dérivé de C-glycoside selon l'invention à raison d'environ 0,0001 % à environ 25 % en poids de matière active par rapport au poids total de la composition, et en particulier d'environ 0,001 % à environ 10 % en poids de C-glycoside par rapport au poids total de la composition, et plus particulièrement d'environ 0,05 à environ 5 % en poids de C-glycoside par rapport au poids total de la composition.

### DERIVES OU ANALOGUES DE L'ACIDE ASCORBIQUE

Le présent texte décrit l'utilisation de l'acide ascorbique (ou vitamine C) ou de l'un de ses analogues ou dérivés.

Selon l'invention, on utilise de l'acide ascorbique.

L'acide ascorbique est généralement sous forme L, car il est habituellement extrait de produits naturels.

En raison de sa structure chimique (alpha-cétolactone) qui le rend très sensible à certains paramètres de l'environnement tels que la lumière, la chaleur et les milieux aqueux, il peut être avantageux d'utiliser l'acide ascorbique sous la forme d'un dérivé ou d'un analogue, par exemple, choisi parmi des esters d'oses de l'acide ascorbique ou des sels métalliques d'acide ascorbique phosphorylé, des sels alcalins, des esters et des sucres.

Les esters d'ose de l'acide ascorbique décrits dans le présent texte sont notamment les dérivés glycosylé, mannosylé, fructosylé, fucosylé, galactosylé N-acétylglucosaminé, N-acétylmuramique de l'acide ascorbique et leurs mélanges et plus spécialement l'ascorbyl-2 glucoside ou 2-O-α-D glucopyranosyl de l'acide L-ascorbique ou encore le 6-O-β-D galactopyranosyl de l'acide L-ascorbique. Ces derniers composés ainsi que leurs procédés de préparation, sont en particulier décrits dans les documents EP-A-0 487 404, EP-A-0 425 066 et JP 05-213 736.

De son côté, le sel métallique d'acide ascorbique phosphorylé peut être choisi parmi les ascorbyl phosphates de métal alcalin, notamment de sodium, les ascorbyl phosphates de métal alcalino-terreux, et les ascorbyl phosphates de métal de transition.

On peut également utiliser des précurseurs d'acide ascorbique tels que les amides d'actif et les dérivés d'ose d'actif, qui font intervenir respectivement comme enzymes des protéases ou des peptidases et des glycosidases pour libérer l'acide ascorbique *in situ.* De tels composés sont décrits dans le brevet EP 0 667 145.

Les dérivés d'ose d'actif sont choisis notamment parmi les dérivés osiques en C₃ à C₆. Ils sont choisis notamment parmi les dérivés glucosylés, mannosylés, fructosylés, fucosylés, N-acétylglucosaminés, galactosylés, N-acétyl-galactosaminés, les dérivés de l'acide N-acétylmuramique, les dérivés d'acide sialique et leurs mélanges.

Les seconds précurseurs d'acide ascorbique peuvent être choisis parmi les dérivés qui sont hydrolysés par d'autres enzymes, par exemple par des estérases, des phosphatases, des sulfatases, etc. On peut choisir par exemple les seconds précurseurs d'actif parmi les phosphates ; les sulfates ; les palmitates ; les acétates ; les propionates ; les férulates et de façon générale les esters alkylés ou acylés d'actif ; les éthers acylés ou alkylés. Les radicaux acylés et alkylés ont en particulier de 1 à 30 atomes de carbone.

En particulier, le second précurseur peut être un ester issu de la réaction avec un acide minéral comme un sulfate ou un phosphate pour réagir avec une sulfatase ou phosphatase au contact de la peau, et le second précurseur un ester acylé ou alkylé issu de la réaction avec un acide organique comme l'acide palmitique, acétique, propionique, nicotinique, 1,2,3-propane tricarboxylique, férulique pour réagir avec une estérase spécifique de la peau.

D'autres dérivés sont décrits par exemple dans le brevet EP 1 430 883.

Les analogues de l'acide ascorbique sont, plus particulièrement, ses sels, notamment alcalins, par exemple l'ascorbate de sodium, ses esters, tels que notamment ses esters acétique, propionique ou palmitique, ou ses sucres, tels que notamment l'acide ascorbique glycosylé.

Selon une variante préférée, il s'agit de l'acide ascorbique.

La quantité efficace en acide ascorbique utilisable selon l'invention est bien entendu celle qui est nécessaire pour obtenir l'effet attendu selon l'invention et en particulier l'effet synergique au regard de son association avec au moins un dérivé C-glycoside selon l'invention.

Pour donner un ordre de grandeur, cette quantité représente préférentiellement de 0,001 % à 20 % du poids total de la composition, préférentiellement de 0,1 % à 15 % du poids total de la composition et avantageusement de 3 % à 10 % du poids total de la composition.

En outre, la composition de l'invention est utilisée pendant un temps suffisant pour obtenir l'effet attendu selon l'invention. Pour donner un ordre de grandeur, cette durée peut être au minimum de 15 jours, mais peut être aussi de plus de 4 semaines, voire de plus de 8 semaines.

Selon un mode de réalisation particulier, l'acide ascorbique peut être associé au(x) dérivé(s) C-glycoside selon l'invention dans un rapport molaire variant de 1 à 10 moles d'acide ascorbique pour 1 mole de dérivé C-glycoside, en particulier dans un rapport molaire variant de 2 à 5 moles d'acide ascorbique pour une mole de dérivé C-glycoside.

Selon un mode de réalisation particulier, la concentration maximum en dérivé C-glycoside est mise en oeuvre à raison d'une concentration inférieure ou égale à 3mM, notamment dans les conditions telles qu'explicitées dans l'exemple.

Selon un autre mode de réalisation, le rapport pondéral entre l'acide ascorbique et le dérivé C-glycoside varie entre 0,6 et 200, par exemple entre 0,6 et 50, entre 50 et 100 ou entre 100 et 200, ou encore entre 1 et 30, notamment entre 1 et 10.

Les deux types de composés tels que définis précédemment peuvent notamment être employés, en association, dans une composition qui comprend un milieu physiologiquement acceptable, notamment dans une composition cosmétique ou pharmaceutique, en particulier dermatologique, qui comprend donc par ailleurs un milieu cosmétiquement ou pharmaceutiquement acceptable.

Le milieu physiologiquement acceptable dans lequel les composés selon invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse.

Selon un mode de réalisation, l'invention a pour objet une composition aqueuse comprenant au moins un dérivé C-glycosidé selon l'invention et au moins de l'acide ascorbique tels que définis dans ce qui précède.

On entend par « composition aqueuse » toute composition comprenant au moins 5 % en poids d'eau par rapport au poids total de la composition et de préférence de 5 à 99 % et encore plus préférentiellement de 20 à 99 % en poids.

Un autre objet de l'invention est une composition anhydre cosmétique et/ou dermatologique comprenant, dans un support physiologiquement acceptable, au moins un dérivé C-glycosylé selon l'invention et au moins de l'acide ascorbique.

On entend par « composition anhydre » toute composition comprenant moins de 5 % d'eau par rapport au poids total de la composition et plus préférentiellement moins de 1 % d'eau.

Pour une application sur la peau, la composition peut avoir la forme notamment d'une solution aqueuse ou huileuse; d'une dispersion du type lotion ou sérum; d'une émulsion de consistance liquide ou semi-liquide du type lait obtenue par dispersion d'une phase grasse dans une phase aqueuse (H/E) ou inversement (E/H) ; d'une suspension ou émulsion de consistance molle du type crème ou gel aqueux ou anhydre ; de microcapsules ou microparticules ; de dispersions vésiculaires de type ionique et/ou non ionique.

Pour une application sur les cheveux, la composition peut être sous forme de solutions aqueuses, alcooliques ou hydroalcooliques; sous forme de crèmes, de gels, d'émulsions, de mousses ; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Ladite phase aqueuse peut comprendre en outre un ou plusieurs solvants organiques tels qu'un alcool en C₁-C₈, notamment l'éthanol, l'isopropanol, le tert-butanol, le n-butanol, des polyols comme la glycérine, le propylène glycol, le butylène glycol, l'isoprène glycol, le polyéthylène glycol et des éthers de polyol.

Lorsque la composition selon l'invention se présente sous la forme d'une émulsion, elle peut éventuellement comprendre en outre un tensioactif, de préférence en une quantité de 0,01 à 30 % en poids par rapport au poids total de la composition. La composition selon l'invention peut également comprendre au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut également comprendre une phase grasse, notamment constituée de corps gras liquides à 25 °C, tels que des huiles d'origine animale, végétale, minérale ou synthétique, volatiles ou non; de corps gras solides à 25 °C tels que des cires d'origine animale, végétale, minérale ou synthétique; de corps gras pâteux; de gommes; de leurs mélanges.

Les huiles volatiles sont généralement des huiles ayant, à 25 °C, une tension de vapeur saturante au moins égale à 0,5 millibar (soit 50 Pa).

Parmi les constituants de la phase grasse, on peut citer :
- les silicones volatiles cycliques ayant de 3 à 8 atomes de silicium, de préférence de 4 à 6.
- les cyclocopolymères du type diméthylsiloxane/méthylalkylsiloxane,
- les silicones volatiles linéaires ayant de 2 à 9 atomes de silicium.
- les huiles volatiles hydrocarbonées, telles que les isoparaffines et notamment l'isododécane et des huiles fluorées.
- les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin ; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales ; des esters d'acides gras ; des alcools ; des acétylglycérides ; des octanoates, décanoates ou ricinoléates d'alcools ou de polyalcools ; des triglycérides d'acides gras ; des glycérides ;
- les huiles fluorées et perfluorées ;
- les gommes de silicones ;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés ; les cires de Candellila, d'Ouricury, de Carnauba, du Japon, le beurre de cacao, les cires de fibres de lièges ou de canne à sucre ; les huiles hydrogénées concrètes à 25 °C, les ozokérites, les esters gras et les glycérides concrets à 25 °C ; les cires de polyéthylène et les cires obtenues par synthèse de Fischer-Tropsch ; des huiles hydrogénées concrètes à 25 °C ; des lanolines ; des esters gras concrets à 25 °C ; les cires de silicone ; les cires fluorées.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques ou pharmaceutiques hydrophiles ou lipophiles, les conservateurs, les antioxydants, les solvants, les parfums, les charges, les pigments, les nacres, les filtres UV, les absorbeurs d'odeur et les colorants. Ces adjuvants, selon leur nature, peuvent être introduits dans la phase grasse, dans la phase aqueuse et/ou dans des sphérules lipidiques.

La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Les compositions cosmétiques ou pharmaceutiques selon l'invention peuvent notamment se présenter sous la forme d'une composition destinée au soin et/ou au traitement des zones ulcérées ou ayant subi un stress ou microstress cutané, notamment généré par une exposition aux UV et/ou la mise en contact avec un produit irritant.

Ainsi, les compositions selon l'invention peuvent notamment se présenter sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps ; une composition anti-rides ou anti-age pour le visage ; une composition matifiante pour le visage ; une composition pour les peaux irritées ; une composition démaquillante ; un lait pour le corps, notamment hydratant éventuellement après-soleil ;
- d'une composition de protection solaire, de bronzage artificiel (autobronzant) ou de soin après-soleil ;
- d'une composition capillaire, et notamment une crème ou un gel protecteur solaire ; une composition de soin du cuir chevelu, notamment anti-chute ou repousse des cheveux;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres.

Les compositions selon l'invention trouvent une application préférée comme composition de soin de la peau du visage, de type anti-rides ou anti-âge, et comme composition de protection solaire ou après-soleil.

La composition utilisée selon l'invention peut en outre contenir d'autres actifs, et notamment au moins un composé choisi parmi : les agents hydratants ; les agents dépigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents myorelaxants ; les agents tenseurs ; les agents anti-pollution et/ou anti-radicalaire, les filtres solaires, et leurs mélanges.

Par « agent hydratant », on entend :
- soit un composé agissant sur la fonction barrière, en vue de maintenir l'hydratation du *stratus corneum,* ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du *stratum corneum,* tel que le tréhalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sébacées tel que la DHEA, ses dérivés 7-oxydés et/ou 17-alkylés, les sapogénines et la vitamine D et ses dérivés.

Par « agent anti-glycation », on entend un composé prévenant et/ou diminuant la glycation des protéines de la peau, en particulier des protéines du derme telles que le collagène. Des exemples d'agents anti-glycation sont les extraits végétaux de la famille des Ericaceae, tels qu'un extrait de myrtille (*Vaccinium angustifolium*) ; l'ergothionéine et ses dérivés ; et les hydroxystilbènes et leurs dérivés, tels que le resvératrol et le 3,3',5,5'-tétrahydroxystilbène.

Des exemples d'inhibiteurs de NO-synthase convenant à une utilisation dans la présente invention comprennent notamment un extrait de végétal de l'espèce *Vitis vinifiera* qui est notamment commercialisé par la société EUROMED sous la dénomination LEUCOCYANIDINES^{®} de raisins extra, ou encore par la société INDENA sous la dénomination LEUCOSELECT^{®}, ou enfin par la société HANSEN sous la dénomination EXTRAIT DE MARC DE RAISIN ; un extrait de végétal de l'espèce *Olea europaea* qui est de préférence obtenu à partir de feuilles d'olivier et est notamment commercialisé par la société VINYALS sous forme d'extrait sec, ou par la société BIOLOGIA & TECHNOLOGIA sous la dénomination commerciale EUROL BT ; et un extrait d'un végétal de l'espèce *Gingko biloba* qui est de préférence un extrait aqueux sec de ce végétal vendu par la société BEAUFOUR sous le nom commercial GINKGO BILOBA EXTRAIT STANDARD.

Parmi les actifs stimulant les macromolécules du derme ou empêchant leur dégradation, on peut citer ceux qui agissent :
- soit sur la synthèse du collagène, tels que les extraits de *Centella asiatica* ; les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale PHYTOKINE^{®} ; et les hormones végétales telles que les auxines et les lignanes.
- soit sur la synthèse d'élastine, tels que l'extrait de *Saccharomyces Cerivisiae* commercialisé par la société LSN sous la dénomination commerciale CYTOVITIN^{®} ; et l'extrait d'algue *Macrocystes pyrifera* commercialisé par la société SECMA sous la dénomination commerciale KELPADELIE^{®} ;
- soit sur la synthèse des glycosaminoglycannes,
- soit sur la synthèse de la fibronectine,
- soit sur l'inhibition des métalloprotéinases (MMP) telles que plus particulièrement les MMP 1, 2, 3, 9. On peut citer : les rétinoïdes et dérivés, les oligopeptides et les lipopeptides, les lipoaminoacides, l'extrait de malt commercialisé par la société COLETICA sous la dénomination commerciale COLLALIFT^{®} ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale FLAVOSTERONE SB^{®}), de trèfle rouge, de lin, de kakkon ou de sauge ;
- soit sur l'inhibition des sérine protéases telles que l'élastase leucocytaire ou la cathepsine G. On peut citer : l'extrait peptidique de graines de légumineuse (*Pisum sativum*) commercialisé par la société LSN sous la dénomination commerciale PARELASTYL^{®} ; les héparinoïdes ; et les pseudodipeptides tels que l'acide {2-[acétyl-(3-trifluorométhyl-phényl)-amino]-3-méthyl-butyrylamino} acétique.

Parmi les actifs stimulant les macromolécules épidermiques, telles que la fillagrine et les kératines, on peut citer notamment l'extrait de lupin commercialisé par la société SILAB sous la dénomination commerciale STRUCTURINE^{®} ; l'extrait de bourgeons de hêtre *Fagus sylvatica* commercialisé par la société GATTEFOSSE sous la dénomination commerciale GATULINE^{®} ; et l'extrait de zooplancton Salina commercialisé par la société SEPORGA sous la dénomination commerciale GP4G^{®}.

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination ELESERYL SH-VEG 8^{®} ou commercialisé par la société SILAB sous la dénomination commerciale RAFFERMINE^{®}) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de *Solanum tuberosum* commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine^{®} ; le beta-sitosteryl sulfate de sodium commercialisé par la société SEPORGA sous la dénomination commerciale PHYTOCOHESINE^{®} ; et l'extrait de maïs commercialisé par la société SOLABIA sous la dénomination commerciale PHYTOVITYL^{®} ; et les lignanes tels que le sécoisolaricirésinol.

Le présent texte décrit également un procédé de traitement cosmétique ou thérapeutique de la peau du corps ou du visage, y compris le cuir chevelu, dans lequel on applique sur la peau une composition cosmétique comprenant une quantité efficace d'au moins un dérivé C-glycoside en association avec de l'acide ascorbique et plus particulièrement telle que définie ci-dessus, à laisser celle-ci en contact puis éventuellement à rincer.

Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs; application d'une lotion pour cuir chevelu sur cheveux mouillés.

L'invention est illustrée plus en détail dans les exemples suivants qui sont présentés à titre illustratif et non limitatif de l'invention.

L'efficacité d'une association conforme à l'invention est testée par dosage du pro-collagène 1. Pour se faire, une composition conforme à l'invention est mise en contact avec des fibroblastes dermiques normaux humains et l'ensemble est laissé incubé 72 heures avant dosage.

Les autres matériaux et le protocole utilisés sont précisés ci-après.

Le dérivé C-glycoside utilisé est le C-β-D-xylopyranoside-2-hydroxy-propane commercialisé sous la dénomination MEROXYL^{®} de CHIMEX. Il se présente sous la forme d'une solution à 30 % en poids en matière active dans un mélange eau/1,2propanediol 60/40.

### Protocole expérimental

- Fibroblastes dermiques humains normaux (NHDF) (R8PF2), obtenus à partir de plastie mammaire.
- Type : pool PF2, utilisé au 8^{ème} passage
- Culture : 37 °C, 5 % CO₂,
- Milieu de culture et d'essai : DMEM (Invitrogen 21969035)
- L-glutamine 2mM (Invitrogen 25030024)
- Pénicilline 50 UI/ml streptomycine 50 µg/ml (Invitrogen 15070063)
- Sérum de veau foetal 10 % (v/v, Invitrogen 10270098).

Les fibroblastes dermiques normaux humains (R8PF2) sont ensemencés en milieu DMEM complet et pré-incubés pendant 24 h à 37 °C et 5 % de CO₂. A 80 % de confluence, le milieu de culture est remplacé par le milieu d'essai DMEM à 10 % SVF contenant ou non (témoin) les produits ou mélanges à l'essai ou la référence (TGF-β). Les cellules sont ensuite incubées à 37 °C pendant 72 heures. Chaque condition expérimentale a été réalisée en triplicata.

Après incubation, les milieux de culture sont prélevés et le dosage procollagène type I est réalisé sur un échantillon de milieu à l'aide d'un kit de dosage Elisa spécifique et selon les directives du fournisseur (Procollagen Type I C-Peptide EIA Kit, Bio-Whittaker MK101). Un test de viabilité au MTT est réalisé sur les tapis cellulaires.

Les résultats sont présentés dans le tableau suivant :

| Traitement | Concentration | Procollagène 1 (ng/ml) | Sd* | N* | %/ témoin | P |
|---|---|---|---|---|---|---|
| Témoin | - | 1922 | 364 | 3 | 100 | <0,01 |
| Dérivé C-glycoside | 1 mM | 1838 | 258 | 3 | 96 | >0,05 |
| acide ascorbique | 0,01 mg/ml | 17052 | 1414 | 3 | 887 | <0,01 |
| Mélange | 1 mM + 0,01 mg/ml | 20159 | 390 | 3 | 1049 | <0,01 |

| | | | | | | |
|---|---|---|---|---|---|---|
| ** N : nombré d'essai* *Sd : Déviation standart* | | | | | | |

Dans ces conditions expérimentales, la quantité de pro-collagène 1 synthétisée et sécrétée par les fibroblastes après 72 heures est correctement détectable. Dans les conditions expérimentales de cet essai (culture 72 h, en milieu DMEM 10 % de SVF), on note que :
- Le dérivé C-glycoside testé à 1 mM ne modifie pas significativement la quantité de pro-collagène 1 synthétisée par les fibroblastes.
- L'acide ascorbique testé à 0,01 mg/ml augmente significativement la synthèse de pro-collagène 1 par les fibroblastes.

Le mélange dérivé C-glycoside/acide ascorbique augmente la synthèse de pro-collagène 1 plus efficacement qu'en présence de l'acide ascorbique seul.

## Revendications

1. Utilisation cosmétique d'une association synergique :
(i) d'au moins un dérivé C-glycoside choisi parmi le C-β-D-xylopyranoside-2-hydroxy-propane, le C-α-D-xylopyranoside-2-hydroxy-propane, et leurs mélanges, avec
(ii) au moins de l'acide ascorbique,
pour traiter et/ou prévenir les signes du vieillissement de la peau du corps ou du visage choisis parmi les rides et/ou ridules, la peau flétrie, le manque d'élasticité et/ou de tonus de la peau, l'amincissement du derme, la dégradation des fibres de collagène, la peau molle et la peau amincie, ou
pour inhiber l'activité des élastases et/ou pour limiter et/ou pour combattre la dégradation des fibres élastiques.

2. Utilisation selon la revendication 1, dans laquelle ledit acide ascorbique et ledit dérivé C-glycoside sont associés dans un rapport molaire variant de 1 à 10.

3. Composition cosmétique et/ou dermatologique comprenant dans un milieu physiologiquement acceptable (i) au moins un dérivé C-glycoside choisi parmi le C-β-D-xylopyranoside-2-hydroxy-propane, le C-α-D-xylopyranoside-2-hydroxy-propane, et leurs mélanges,, en association avec (ii) au moins de l'acide ascorbique.

4. Composition selon la revendication 3, ladite composition étant anhydre.

5. Composition selon la revendication 3, ledit milieu physiologiquement acceptable contenant une phase aqueuse.

6. Composition selon l'une quelconque des revendications 3 à 5, comprenant de 0,001 à 20 % en poids, notamment de 0,1 à 15 % en poids, de préférence de 3 à 10 % en poids, d'acide ascorbique par rapport au poids total de la composition.

7. Procédé de traitement cosmétique de la peau du corps ou du visage, y compris le cuir chevelu, dans lequel on applique sur la peau une composition cosmétique telle que définie dans l'une des revendications 3 à 6.

## Patentansprüche

1. Kosmetische Anwendung einer synergistischen Kombination:
(i) von mindestens einem C-Glycosidderivat, ausgewählt aus C-ß-D-Xylopyranosid-2-hydroxypropan, C-α-D-xylopyranosid-2-hydroxypropan, und ihren Gemischen, mit
(ii) mindestens Ascorbinsäure,
zur Behandlung und/oder Vorbeugung der Anzeichen der Alterung der Haut des Körpers oder des Gesichts, ausgewählt aus Falten und/oder Fältchen, welker Haut, mangelnder Elastizität und/oder Spannung der Haut, Dünnerwerden der Lederhaut, Abbau der Kollagenfasern, schlaffer Haut und dünner Haut, oder
zur Hemmung der Aktivität der Elastasen und/oder zur Begrenzung und/oder Bekämpfung des Abbaus der elastischen Fasern.

2. Anwendung nach Anspruch 1, wobei die Ascorbinsäure und das C-Glycosidderivat in einem von 1 bis 10 variierenden Molverhältnis kombiniert sind.

3. Kosmetische und/oder dermatologische Zusammensetzung, umfassend in einem physiologisch verträglichen Medium (i) mindestens ein C-Glycosidderivat, ausgewählt aus C-ß-D-Xylopyranosid-2-hydroxypropan, C-α-D-xylopyranosid-2-hydroxypropan und ihren Gemischen, in Kombination mit (ii) mindestens Ascorbinsäure.

4. Zusammensetzung nach Anspruch 3, wobei die Zusammensetzung wasserfrei ist.

5. Zusammensetzung nach Anspruch 3, wobei das physiologisch verträgliche Medium eine wässrige Phase enthält.

6. Zusammensetzung nach einem der Ansprüche 3 bis 5, umfassend von 0,001 bis 20 Gew.-%, insbesondere von 0,1 bis 15 Gew.-%, vorzugsweise von 3 bis 10 Gew.-% Ascorbinsäure, bezogen auf das Gesamtgewicht der Zusammensetzung.

7. Kosmetisches Behandlungsverfahren der Haut des Körpers oder des Gesichts, darunter die Kopfhaut, wobei auf die Haut eine kosmetische Zusammensetzung, wie in einem der Ansprüche 3 bis 6 definiert, aufgebracht wird.

## Claims

1. Cosmetic use of a synergistic combination:
(i) of at least one C-glycoside derivative selected from among C-β-D-xylopyranoside-2-hydroxy-propane, C-α-D-xylopyranoside-2-hydroxy-propane and mixtures thereof, with
(ii) at least ascorbic acid,
for treating and/or preventing signs of skin ageing of the body or face selected from among wrinkles and/or fine lines, wizened skin, lack of skin elasticity and/or tonicity, thinning of the dermis, degradation of collagen fibers, flaccid skin and thinned skin, or
for inhibiting the activity of elastases and/or for limiting and/or combating degradation of elastic fibers.

2. The use according to claim 1, wherein said ascorbic acid and said C-glycoside derivative are combined in a molar ratio varying from 1 to 10.

3. A cosmetic and/or dermatological composition comprising in a physiologically acceptable medium (i) at least one C-glycoside derivative selected from among C-β-D-xylopyranoside-2-hydroxy-propane, C-α-D-xylopyranoside-2-hydroxy-propane and mixtures thereof, in combination with (ii) at least ascorbic acid.

4. The composition according to claim 3, said composition being anhydrous.

5. The composition according to claim 3, said physiologically acceptable medium containing an aqueous phase.

6. The composition according to any of claims 3 to 5, comprising from 0.001 to 20% by weight, notably from 0.1 to 15% by weight, preferably from 3 to 10% by weight, of ascorbic acid based on the total weight of the composition.

7. A cosmetic treatment method of the skin of the body or the face, including the scalp, wherein a cosmetic composition as defined in one of claims 3 to 6 is applied on the skin.
